# EUROPEAN PATENT APPLICATION

(11) **EP 0 769 298 A2**
(43) Date of publication of application: **23.04.1997**
(21) Application number: 96202843.7
(22) Date of filing: 11.10.1996
(51) Int. Cl.: A61K 35/74

(54) **Pharmaceutical compositions for topical use containing hyaluronic acid and Döderlein's bacillus for the prevention and treatment of vaginal diseases**

(30) Priority: 16.10.1995 IT MI952117
(71) Applicant: LABORATORI BALDACCI Spa, 56100 Pisa (IT)
(72) Inventor: Baldacci, Massimo c/o LABORATORI BALDACCI SPA, 56100 Pisa (IT)
(74) Representative: Dragotti, Gianfranco

(57) **Abstract**

The present invention relates to the topical use of hyaluronic acid and Döderlein's bacillus in the prevention and therapeutic treatment of vaginal diseases caused by harmful agents, by a reduced oestrogen supply and/or by a changed equilibrium of the vaginal bacterial flora.

## Description

The present invention relates to the topical use of hyaluronic acid and Döderlein's bacillus in the prevention and therapeutic treatment of vaginal diseases caused by harmful agents, by a reduced oestrogen supply and/or by a changed equilibrium of the vaginal bacterial flora.

Vaginal bacterial flora is an example of a composite ecosystem, knowledge of which is important in the understanding of the aetiology of infectious diseases of the female genital organs.

A particular feature of normal vaginal flora under normal conditions is the fact that only a relatively small number of bacterial and fungal strain are capable of colonising the vagina.

From 5 to 10 different types of micro-organism of which the concentration can vary from a few units to 10⁸⁻⁹ CFU (colony-forming units) can be identified in every vagina, including the clinically normal vagina (H.A. Hammil: Obstetric and Gynecologic Infections 6,(2),329,1989).

The vagina is in fact the seat of a biological process which maintains the equilibrium of the purity of the bacterial flora and which has the task of creating an environment which is unsuitable for the establishment of pathogens (D.A. Eschanbach: Obstetric and Gynecol. Clin.. of North Am. 16(3),593,1989).

Döderlein's bacillus plays a fundamental part in this self-defence process and, by converting the glycogen of the epithelial cells into lactic acid, reduces the vaginal pH to values of from 3.8 to 4.4, thus providing an optimum acidic environment for its growth and at the same time preventing the survival of the pathogens which reproduce at higher pH values (Jay A. Swenberg: Comprehensive Therapy 15,(8), 47-53,1989; B. Sandler. Lancet II, 791-792, 1979).

It has also been recognised for some time that the acidity of the vaginal tissue plays a role, although not the only one, in this self-defence mechanism and that there is a close correlation between the pH value and changes in the microbial flora (S.Y. Drakem B.A. Evanc, A. Gerken: Brit.J.Vener.Dis. 56,107-110, 1980).

In fact, the mechanism which determines the physico-chemical equilibrium is started by the action of circulating oestrogens which act selectively on the receptors in the vaginal tissue and give rise to a more favourable development of Döderlein's bacillus, the growth of which then fixes the pH at a more acidic and optimum level for the lactobacillus.

This physico-chemical equilibrium in the vagina, that is to say, the presence of acidity and of pure saprophytic flora, may change when one of the factors discussed above is missing. For example, this may already be observed in normal physiological conditions: before puberty, when the ovarian function is low and the vaginal epithelium is low in glycogen, the presence of a slight acidity (pH = 6) is observed, which condition is till sufficiently protective in the absence of foreign pathogenic agents; however, if foreign pathogenic agents occur, the impuberal vagina has a very limited defensive power which is why an infectious process with considerable subjective and objective symptomatology may easily become established. In addition, in the immediately post-menstrual period, when the production of oestrogens is low and the menstrual blood has temporarily destroyed the state of acidity of the vaginal canal, infections may increasingly occur.

Also, after the menopause, the low level of glycogen in the vaginal mucosa is the main cause of senile vaginitis, the severity of which is partly attenuated by the state of atrophy of the vagina.

Thus, knowing the self-defence mechanism of the vaginal mucosa, whenever a pathological process occurs it is necessary to re-establish the flora and the associated physico-chemical equilibrium, aiming each time, wherever possible, to detect the factor responsible for breaking the chain which governs the physiology thereof.

The usual treatment for vaginal diseases is based on antisepsis; the use of bactericidal substances, however, although involving an improvement in the symptomatology, never provides protection against possible relapses because it does not allow normal physiological conditions to be restored in the vagina, that is to say, suitable acidity and a true and non-pathogenic bacterial flora.

Antibiotic or sulphonamide treatment causes harmful effects owing to the destruction of the normal vaginal flora, both in the case of local application and in the case of general application.

It is known that, with Döderlein's bacilli, the natural vaginal bacterial flora can be reconstructed by the topical application of a concentrated quantity of lactobacilli in order to create environmental conditions which prevent the survival of pathogenic agents and reduce the possibility of their becoming established.

Döderlein's bacillus thus offers the possibility of reconstructing a habitat which is unfavourable to the establishment of germs, by reducing the number of strains thereof and is a useful complement to chemotherapeutic treatment in the forms described for restoring the normal physiological conditions of the vaginal environment at the end of the treatment.

In addition to Döderlein's bacillus, hyaluronic acid, which is a constituent of the vaginal epithelium and the integrity of which may determine the deterioration of the vaginal disease, contributes greatly to this defence mechanism. Biochemical study of the cervico-vaginal tissue demonstrates the role played by hyaluronic acid and by other glycosaminoglycans in the formation and function of the vaginal mucus which, as is known, owing to its physical properties, such as adhesiveness, viscosity and gel formation, exhibits a general anti-bacterial activity.

The vagina, under normal physiological conditions and in the presence of normal secretion, has an acid pH of from 3.5 to 5; hyaluronic acid, given its acidic character, also contributes to the maintenance of these pH values and to the formation of a quantity of physiological mucus.

It is clear from the above considerations that both Döderlein's bacillus and hyaluronic acid principally and particularly have a restorative function, once the main chemotherapeutic attack has been completed, and when pathological conditions exist.

It has now been found, and forms the main subject-matter of the present invention, that the concomitant presence of these two active ingredients in a single formulation suitable for topical treatment assumes the characteristics of a drug capable of combating and overcoming these pathological conditions. More specifically, therefore, the present invention relates to the topical use of hyaluronic acid and Döderlein's bacillus in the treatment of the states of:
- aspecific phlogosis of the female genital organs;
- modification of the vaginal ecosystem caused by infection with pathogens and harmful agents;
- dystrophy and atrophy owing to a lack of oestrogen hormone and senile vaginitis.

The topical application may involve globuli, pessaries, creams, ointments, lotions, salves and any other type of application formulation for topical use in the vagina, containing the two active ingredients in the following dosages:
- hyaluronic acid: from 2 to 50 mg per unit dose;
- Döderlein's bacillus: from 100 to 200 mg of lyophilised powder per unit dose.
   As is demonstrated by the experimental results obtained, the presence of the two active ingredients has a two-fold beneficial effect: hyaluronic acid acts by restoring the vaginal mucus content and by repairing the tissue damage caused by harmful agents or by oestrogen deficiency; Döderlein's bacillus maintains the bacterial flora in equilibrium by creating an environment which is unsuitable for the establishment of pathogens.

When conditions of bacterial superinfection promoted by poor hygiene are established, or when the cell glycogen decreases owing to states of reduced oestrogen supply (childhood and menopause), the activity of Döderlein's bacillus is no longer sufficient to keep the vaginal surroundings acidic. It is possible that the infection of the vagina will also spread to the ovaries and to the uterus, thus causing adnexa and endometritis, which are definitely more difficult to combat therapeutically.

The use of antibiotics and sulphonamides is not appropriate because these substances act indiscriminately both on pathogens and on Döderlein's bacillus, further increasing vaginal alkalinity.

However, the use of vaginal lavages, pessaries, globuli or vaginal salves based on Döderlein's bacillus and on hyaluronic acid makes it possible, by means of a physiological mechanism, to control phlogistic diseases efficiently and, at the same time, to reduce the concentration of pathogens.

The compositions according to the present invention were the subject of preliminary pharmacological tests using the two active ingredients and a mixture thereof both in lyophilised form and in the form of a pessary containing the customary excipients and carriers.

### Stability studies

The stability studies were carried out on the end product and on the pharmaceutical product.
a) End product: 80 mg of lyophilised Döderlein's bacillus + 10 mg of hyaluronic acid, contained in glass vials under vacuum at a temperature of 20°C.
Titre expressed in live germs/mg of lyophilisate.

| Time | 0 | 30 days | 90 days | 180 days |
|---|---|---|---|---|
| | 3.10⁶ | 3.10⁶ | 2.8.10⁶ | 3.10⁶ |

b) Pharmaceutical product: formulated as pessaries each containing 40 mg of Döderlein's bacillus + 5 mg of hyaluronic acid in glass vials under vacuum at a temperature of 20°C.

| Time | 0 | 30 days | 90 days | 180 days |
|---|---|---|---|---|
| | 1.5.10⁷ | 2.10⁷ | 1.6.10⁷ | 1.10⁷ |

### Activity tests

The microscopic picture of the vaginal fluid can be changed by local inflammatory processes and by diseases. The following four grades were established in connection with the microscopic characteristics which may be exhibited by the vaginal smear:

| | |
|---|---|
| Grade I (strongly acidic pH) | Desquamated vaginal cells, Döderlein's bacillus and mucus filaments are observed under the microscope. |
| Grade II (acidic pH) | Microscopic picture identical to that above but with the presence of pathogens and leucocytes in addition to Döderlein's bacillus |
| Grade III (alkaline pH) | No Döderlein's bacilli. Pathogens and leucocytes present. |
| Grade IV (strongly alkaline pH) | No Döderlein's bacilli. A very large number of pathogens and leucocytes are present. |

Grades I and II may be regarded as normal while grades III and IV are the manifestation of a pathological state in which the phlogosis is accompanied by an infection caused by germs such as Trichomonas, Streptococcus, Staphylococcus, Escherichia coli, fungi, etc. The phlogosis, which may initially be entirely asymptomatic, soon takes root and causes imbalance and tissue dystrophy.

20 women with a vaginal picture of type III and IV were treated with Döderlein's bacillus and hyaluronic acid based pessaries of the type indicated above. The dosage used was the following: one pessary in the morning and one in the evening for a period of 10 days.

Subjective clinical symptoms (itching, burning, pain) and objective signs of phlogosis (congestion, oedema) were observed. The intensity of the symptomatology was always expressed by a score varying from 0 (absent) to 3 (intense).

Each patient also gave a vaginal smear with bacteriological examination and measurement of the vaginal pH. At the end of the experiment all the data collected were subjected to statistical analysis using Student's "t" test fro paired data.

**Table 1**

| Clinical symptoms before and after treatment with Döderlein's bacillus and hyaluronic acid based pessaries | | | | | | |
|---|---|---|---|---|---|---|
| Patient No. | Itching | | Burning | | Pain | |
| | Before | After | Before | After | Before | After |
| 1 | 3 | 1 | 2 | 1 | 1 | 0 |
| 2 | 3 | 2 | 2 | 1 | 1 | 0 |
| 3 | 1 | 0 | 2 | 1 | 1 | 0 |
| 4 | 1 | 1 | 2 | 2 | 0 | 0 |
| 5 | 2 | 2 | 1 | 1 | 1 | 1 |
| 6 | 1 | 0 | 2 | 2 | 2 | 2 |
| 7 | 3 | 2 | 2 | 1 | 1 | 0 |
| 8 | 2 | 1 | 1 | 1 | 1 | 1 |
| 9 | 2 | 2 | 2 | 1 | 2 | 1 |
| 10 | 3 | 2 | 3 | 2 | 2 | 1 |
| 11 | -2 | 1 | 1 | 0 | 1 | 0 |
| 12 | 2 | 0 | 2 | 0 | 1 | 1 |
| 13 | 1 | 0 | 1 | 1 | 0 | 0 |
| 14 | 2 | 1 | 2 | 1 | 1 | 1 |
| 15 | 1 | 0 | 2 | 1 | 1 | 0 |
| 16 | 1 | 1 | 1 | 0 | 2 | 1 |
| 17 | 2 | 1 | 2 | 0 | 1 | 0 |
| 18 | 1 | 0 | 1 | 1 | 1 | 0 |
| 19 | 3 | 3 | 3 | 1 | 2 | 2 |
| 20 | 1 | 1 | 1 | 0 | 1 | 0 |

**Table 2**

| Objective signs before and after treatment with Döderlein's bacillus + hyaluronic acid based pessaries | | | | |
|---|---|---|---|---|
| Patient No. | Congestion | | Oedema | |
| | Before | After | Before | After |
| 1 | 2 | 0 | 1 | 0 |
| 2 | 3 | 0 | 3 | 1 |
| 3 | 2 | 1 | 1 | 0 |
| 4 | 2 | 0 | 1 | 0 |
| 5 | 3 | 2 | 3 | 1 |
| 6 | 2 | 1 | 2 | 1 |
| 7 | 0 | 0 | 1 | 0 |
| 8 | 3 | 1 | 2 | 1 |
| 9 | 0 | 0 | 0 | 0 |
| 10 | 2 | 1 | 2 | 2 |
| 11 | 3 | 2 | 3 | 1 |
| 12 | 1 | 0 | 1 | 0 |
| 13 | 3 | 3 | 2 | 2 |
| 14 | 3 | 0 | 1 | 0 |
| 15 | 2 | 0 | 2 | 2 |
| 16 | 1 | 0 | 2 | 1 |
| 17 | 1 | 1 | 2 | 1 |
| 18 | 2 | 0 | 3 | 0 |
| 19 | 2 | 1 | 3 | 1 |
| 20 | 0 | 0 | 1 | 1 |

**Table 3**

| pH measurement carried out on the vaginal smear before and after treatment with Döderlein's bacillus + hyaluronic acid based pessaries | | |
|---|---|---|
| Patient No. | pH | |
| | Before | After |
| 1 | 5.8 | 4.5 |
| 2 | 6.0 | 4.5 |
| 3 | 6.0 | 5.0 |
| 4 | 6.8 | 5.0 |
| 5 | 6.7 | 5.3 |
| 6 | 6.0 | 4.7 |
| 7 | 5.5 | 4.5 |
| 8 | 5.8 | 5.3 |
| 9 | 6.0 | 5.6 |
| 10 | 5.8 | 5.2 |
| 11 | 5.2 | 4.7 |
| 12 | 6.0 | 4.5 |
| 13 | 5.9 | 4.3 |
| 14 | 5.5 | 5.0 |
| 15 | 5.5 | 5.2 |
| 16 | 5.8 | 4.0 |
| 17 | 5.0 | 4.2 |
| 18 | 5.2 | 4.2 |
| 19 | 5.5 | 4.6 |
| 20 | 6.0 | 5.1 |

**Table 4**

| Pathogens and Döderlein's bacillus before and after treatment with pessaries | | | | | | |
|---|---|---|---|---|---|---|
| Patient No. | Gram + | | Gram + | | Döderlein's bacillus | |
| | Before | After | Before | After | Before | After |
| 1 | 10 | 10 | 10⁴ | 10 | 10³ | 10⁴ |
| 2 | 10² | 10² | 10⁷ | 10² | 0 | 10² |
| 3 | 0 | 0 | 10⁴ | 0 | 10⁵ | 10⁴ |
| 4 | 10 | 10 | 10⁴ | 0 | 10 | 10⁴ |
| 5 | 10² | 0 | 10⁴ | 0 | 0 | 0 |
| 6 | 10 | 0 | 0 | 0 | 10³ | 10⁴ |
| 7 | 10² | 10 | 10⁵ | 10² | 0 | 10³ |
| 8 | 10 | 10 | 10² | 0 | 10² | 10³ |
| 9 | 10² | 10 | 10⁵ | 10 | 10³ | 10⁵ |
| 10 | 10² | 10 | 0 | 0 | 10² | 10³ |
| 11 | 10 | 10 | 10³ | 0 | 10 | 10³ |
| 12 | 0 | 0 | 10³ | 0 | 10² | 10⁴ |
| 13 | 10² | 10 | 10² | 0 | 10² | 10⁶ |
| 14 | 10 | 0 | 10³ | 0 | 10 | 10⁴ |
| 15 | 10⁶ | 10³ | 0 | 0 | 0 | 10 |
| 16 | 0 | 0 | 10³ | 0 | 10² | 10³ |
| 17 | 10² | 10² | 10⁵ | 0 | 0 | 10⁴ |
| 18 | 10² | 10² | 0 | 0 | 10³ | 10⁴ |
| 19 | 10⁴ | 0 | 10⁴ | 0 | 0 | 10⁴ |
| 20 | 10² | 0 | 10⁴ | 10 | 0 | 10⁴ |

The regression and disappearance of the phlogosis with restoration of normal vaginal acidity are demonstrated by the experimental results obtained, confirming the efficacy of the medicament administered topically in vaginal globuli.

## Claims

1. Topical use of a composition containing in admixture, as active ingredients, Döderlein's bacillus and hyaluronic acid in the prevention and treatment of vaginal diseases exhibiting alteration of the flora and increased pH and dystrophy of the vaginal tissue.

2. Topical use according to claim 1, characterised in that the active ingredients are formulated as globuli, creams ointments, lavages, lotions and salves containing from 0.1 to 5% by weight of the active ingredients taken together.

3. Topical use according to claim 1, characterised in that the content of hyaluronic acid varies from 2 to 50 mg and that of Döderlein's bacillus varies from 20 to 500 mg for each single unit dose.

4. Topical use according to claim 1, characterised in that the composition is used for the prevention and treatment of states of dystrophy and atrophy where there is an oestrogen deficiency, senile vaginitis, alteration in the vaginal ecosystem and aspecific vaginitis with infection also of the tissue of the uterus and the ovaries.
